# EUROPÄISCHE PATENTSCHRIFT

(11) **EP 3 630 422 B1**
(45) Veröffentlichungstag und Bekanntmachung des Hinweises auf die Patenterteilung: **06.09.2023**
(21) Anmeldenummer: 18728072.2
(22) Anmeldetag: 22.05.2018
(51) Int. Cl.: B25J 13/02, A61B 8/00, B25J 19/06, A61B 34/30, A61B 90/00, B25J 13/08

(54) **MANIPULATORSYSTEM MIT EINGABEMITTEL ZUR KRAFTREDUKTION**
MANIPULATOR SYSTEM WITH INPUT MEANS FOR FORCE REDUCTION
SYSTÈME DE MANIPULATION COMPRENANT UN MOYEN DE SAISIE POUR LA RÉDUCTION DE FORCES

(30) Priorität: 30.05.2017 DE 102017209034
(43) Veröffentlichungstag der Anmeldung: 08.04.2020
(73) Patentinhaber: KUKA Deutschland GmbH, 86165 Augsburg (DE)
(72) Erfinder: KELLER, Henrik, 40219 Düsseldorf (DE); VIRGA, Salvatore, 81543 München (DE)
(74) Vertreter: Oelke, Jochen
(86) Internationale Anmeldenummer: PCT/EP2018/063370
(87) Internationale Veröffentlichungsnummer: WO 2018/219717

(56) Entgegenhaltungen:
- EP-A1- 2 412 406
- WO-A1-2017/020081
- DE-A1-102007 046 700
- DE-U1-202016 106 554
- US-A1- 2007 205 785

## Beschreibung

### 1. Technischer Bereich

Die vorliege Erfindung betrifft ein Manipulatorsystem als auch ein Verfahren zum Steuern eines Manipulators.

### 2. Technischer Hintergrund

Manipulatoren, und insbesondere Roboter, sind frei- programmierbare Handhabungsgeräte, die universell eingesetzt werden können. Ein Manipulator kann dabei mehrere Achsen haben, die unabhängig voneinander mittels entsprechender Antriebe, wie beispielsweise Servomotoren, bewegbar sind. Dadurch kann der Manipulator verschiedene Posen bzw. verschiedene Konfigurationen einnehmen, um etwa ein Instrument an eine bestimmte Position im Raum zu bewegen, oder um einen bestimmten Prozess durchzuführen.

Beim Einsatz in der Medizintechnik kann ein Manipulator beispielsweise verwendet werden, um eine Behandlung, wie etwa eine Untersuchung oder einen Eingriff, durchzuführen. Hierzu kann der Manipulator ein entsprechendes Instrument bzw. medizinisches Instrument führen, wie beispielsweise ein Skalpell oder einen Ultraschallkopf. Der Manipulator kann während des Eingriffs bzw. während der Untersuchung, oder allgemein während der Behandlung, eine Trajektorie oder eine Bahn abfahren, wobei die einzelnen Punkte der Bahn durch ein Manipulator- bzw Roboterprogramm oder eine Bahnplanung vorgegeben sein können.

Zur Programmierung eines Manipulator- bzw. Roboterprogramms müssen die Positionen einzelner Bahnpunkte, welche von dem Manipulator angefahren werden sollen, erfasst bzw. vorgegeben werden. Bei der sogenannten Offline-Programmierung können beispielsweise mittels entsprechender Modelle oder Simulations-Tools einzelne Bahnpunkte generiert werden. Bei der sogenannten Online-Programmierung erfolgt die Programmierung direkt am Manipulator. So kann auch eine Bahnplanung live vorgegeben werden, in dem ein Bediener den Manipulator mittels eines entsprechenden Handhabungsgeräts steuert.

Beim Einsatz von Manipulatoren in der Medizintechnik existiert jedoch ein großes Vertrauensproblem. Viele Patienten und auch Ärzte vertrauen einem Manipulatorsystem nicht in einem solchen Maße, dass sie diese bedingungslos für einen kritischen Behandlungsschritt einsetzen würden. Aus diesem Grund sind die meisten Manipulatorsysteme in der Medizintechnik lediglich teleoperierend oder semi-autonom ausgestaltet. Ein semi-autonom ausgestalteter Manipulator kann dabei beispielsweise lediglich unterstützend zur Verfügung stehen, in dem er eine Instrumentorientierung hält, während der Bediener bzw. Arzt weiterhin die Kontrolle über den Kontakt zwischen dem Instrument und dem Patienten behält. Beispielsweise kann die Orientierung eines Skalpells durch den Manipulator vorgegeben werden, während der Vorschub durch den Arzt bestimmt wird.

Bei chirurgischen Eingriffen ist der Patient üblicherweise sediert, sodass sein Vertrauensproblem während des Eingriffs selbst kein Problem darstellt. Bei Applikationen, bei denen der Patient jedoch nicht sediert ist und mit dem Manipulator in Berührungskontakt tritt, stellt das mangelnde Vertrauen jedoch ein großes Problem dar. Eine solche Situation kann beispielsweise vorliegen, wenn durch einen Manipulator ein Ultraschallkopf entlang des Patientenkörpers geführt werden soll. Bei der Untersuchung durch Ultraschall muss oft ein für den Patienten unangenehm hoher Druck mit dem Ultraschallkopf aufgebracht werden, da sonst weiter im Körperinneren liegende Organe nicht gut sichtbar sind. Wenn ein Arzt den Ultraschallkopf führt, kann der Patient bei zu hohem Druck mit dem Arzt reden und diesen Bitten, den Druck zu reduzieren. Wenn ein Manipulator den Ultraschallkopf führt, bietet sich jedoch eine solche intuitive Möglichkeit nicht.

Eine andere unangenehme Situation kann sich ergeben, wenn der Ultraschallkopf trotz sorgfältiger Bahnplanung in falscher oder ungünstiger Orientierung gegen den Patienten gedrückt bzw. gepresst wird, oder wenn nicht genügend Ultraschall-Gel als Gleitmittel auf die Patientenhaut aufgetragen wurde, oder wenn der Ultraschallkopf gegen eine vorher unbekannte (zum Beispiel krankheitsbedingte) sensible Stelle am Patientenkörper presst.

Die US Patentschrift US 6,267,737 B1 beschreibt ein Robotersystem, welches verwendet werden kann, um wiederholt konzentrierten Druck auf bestimmte Muskeln in einem Patientenkörper auszuüben. Dabei kann ein maximaler Druck eingestellt werden, bei dessen Überschreitung der Roboter von dem Patientenkörper entfernt wird. Der Druck wird damit mechanisch aufgehoben, und letztendlich die Behandlung abgebrochen.

Die internationale Veröffentlichung WO 2017/020081 A1 offenbart ein teleoperiertes Robotersystem bei dem eine Messsonde über die Körperoberfläche eines Patienten bewegt werden kann. Die Messsonde wird dabei über ein Dämpfungselement am Flansch des Roboters befestigt, so dass Unebenheiten oder Bewegungen des Patienten durch das Dämpfungselement abgefedert werden.

Es ist eine Aufgabe der vorliegenden Erfindung, ein Manipulatorsystem bereitzustellen, welches die oben genannten Probleme, zumindest teilweise, überwindet. Dabei ist es insbesondere eine Aufgabe der vorliegenden Erfindung, es einem Bediener oder Patienten zu ermöglichen, ein gewisses Maß an Kontrolle über den Manipulator zu gewinnen, um dadurch eine Vertrauenssteigerung zu erzielen.

Diese und weitere Aufgaben, die aus der folgenden Beschreibung für den Fachmann ersichtlich werden, werden durch ein Manipulatorsvstem gemäß Anspruch 1 und ein Verfahren zum Steuern eines Manipulators gemäß Anspruch 13, gelöst.

### 3. Inhalt der Erfindung

Die vorliegende Erfindung betrifft ein Manipulatorsystem. Das Manipulatorsystem umfasst dabei einen Manipulator, der beispielsweise als mehrachsiger Gelenkarraroboter ausgestaltet sein kann. Der Manipulator ist dabei eingerichtet zum Führen eines Instruments. Das Instrument kann dabei beispielsweise einen Endeffektor des Manipulators bilden, und kann dabei das letzte Element einer kinematischen Kette des Manipulators sein. Das Instrument kann beispielsweise ein Ultraschallmesskopf sein, oder aber auch z.B. ein Rasierer oder ein anderes Instrument für kosmetische Anwendungen. Die Erfindung ist jedoch nicht auf ein bestimmtes Instrument beschränkt. Generell kann das Instrument eingerichtet sein, eine Behandlung bzw. Untersuchung oder Eingriff an einem menschlichen Körper bei direktem Kontakt mit diesem Körper durchzuführen.

Das Manipulatorsystem umfasst ferner ein Steuermittel, welches eingerichtet ist, den Manipulator derart anzusteuern, dass das Instrument mit einer Anpresskraft gegen den menschlichen Körper gepresst wird. Das Steuermittel kann als Steuerung ausgestaltet sein, welche im Manipulator implementiert oder separat zu diesem bereitgestellt sein kann. Der menschliche Körper kann dabei beispielsweise ein Patient sein. Vorzugsweise kann das Instrument gemäß einer Bahnplanung gegen den menschlichen Körper gepresst werden. Die Bahnplanung kann dabei vorab bereitgestellt sein, oder sich im Zuge einer manuellen, direkten Ansteuerung des Manipulators durch einen Anwender ergeben. Die Anpresskraft folgt aus der Berührung zwischen dem Instrument und dem Körper. Diese Anpresskraft kann dabei beispielsweise einen Newton betragen. Die Anpresskraft kann aber auch je nach Anwendungsfall deutlich über oder unterhalb von einem Newton liegen. Beispielsweise kann das Steuermittel eingerichtet sein, den Manipulator derart anzusteuern, dass ein Ultraschallkopf mit einer solchen Anpresskraft gegen einen Patienten gepresst wird, dass eine Ultraschalluntersuchung durchgeführt werden kann.

Das Manipulatorsystem umfasst ferner ein Kraftreduktion-Eingabemittel, welches separat zum Manipulator bereitgestellt ist. Das Kraftreduktion-Eingabemittel kann beispielsweise als Eingabevorrichtung ausgestaltet sein, welche separat zum Manipulator vorliegt. Das Kraftreduktion-Eingabemittel kann dabei mit dem Steuermittel direkt oder indirekt kommunizieren, beispielsweise über eine kabellose oder über eine kabelgebundene Schnittstelle. Das Kraftreduktion-Eingabemittel ist von einem Bediener betätigbar zur Reduktion der Anpresskraft. Der Bediener kann dabei jene Person sein, gegen welche das Instrument gepresst wird (der menschliche Körper), beispielsweise der Patient, oder aber auch ein Arzt oder eine andere Person, welche den Vorgang überwacht.

Das Steuermittel ist dabei ferner eingerichtet, den Manipulator derart anzusteuern, dass die Anpresskraft auf einen Betrag bzw. Wert reduziert wird, welcher größer Null ist, basierend auf einer entsprechenden Betätigung des Kraftreduktion-Eingabemittels. Die Anpresskraft wird dabei reduziert, jedoch wird der Kontakt zwischen dem Instrument und dem menschlichen Körper nicht aufgehoben, da die resultierende Anpresskraft größer Null bleibt.

Mittels des Kraftreduktion-Eingabemittels kann der Bediener somit die vom Manipulator ausgeübte Kraft reduzieren. Die Steuerung kann auf eine solche Vorgabe vom Bediener reagieren und den Manipulator derart ansteuern, dass gezielt die Anpresskraft reduziert wird, ohne notwendigerweise einen Abbruch der Behandlung zu veranlassen. Der Einsatz des Manipulators, bzw. die Untersuchung, wird dabei nicht zwangsläufig abgebrochen, sondern kann mit der verringerten Anpresskraft fortgeführt werden. Vorzugsweise erfolgt dieses Reduzieren der Anpresskraft, während die Bahnplanung abgefahren wird. Der Bediener kann somit die Kraftreduktion interaktiv durchführen, also unmittelbar während der Untersuchung. Vorteilhafterweise kann der Behandlungsschritt mit der reduzierten Anpresskraft fortgeführt werden.

Der Fachmann versteht, dass das Maß der Anpresskraft, die vom Manipulator auf den menschlichen Körper ausgeübt wird, je nach Anwendungsart keine schweren Verletzungen des Körpers hervorrufen darf. Vorzugsweise kann die Anpresskraft im Bereich von 0,01 N bis 100 N liegen, weiter vorzugsweise im Bereich von 0,05 bis 30N, weiter vorzugsweise im Bereich von 0,1 N bis 10 N liegen.

Gemäß der Erfindung ist das Manipulatorsystem eingerichtet, während des Pressens Messwerte mittels des Instruments zu erfassen. Vorzugsweise kann das Instrument zur Bildgebung und insbesondere zur Tomografie eingerichtet sein. Somit kann das Manipulatorsystem entsprechende Bilddaten erfassen. Das Manipulatorsystem umfasst dabei ferner ein Messwert-Auswertemittel, welches eingerichtet ist eine Qualität der Messwerte zu erfassen. Beispielsweise kann mit Hilfe einer Metrik die Qualität von erfassten Bildern bestimmt werden. Eine solche Metrik könnte durch Confidence Maps, ROI-Tracking oder andere Techniken realisiert werden, mit denen die (vollständige) Sichtbarkeit der jeweiligen Anatomie festgestellt werden kann. Das Steuermittel ist ferner eingerichtet den Manipulator basierend auf der erfassten Qualität der Messwerte anzusteuern. Beispielsweise kann die Anpresskraft ohne Eingabe durch den Bediener reduziert werden, wenn es die Qualität der Messwerte bereits erlaubt.

Vorzugsweise ist das Kraftreduktion-Eingabemittel eine Benutzer-Schnittstelle. Das Manipulatorsystem ist somit um eine solche Schnittstelle für beispielsweise einen Patienten erweitert. Über diese Schnittstelle kann der Patient immer den aktuellen vom Manipulatorsystem ausgeübten Druck regulieren, und kann diesen interaktiv verringern. Vorzugsweise umfasst das Kraftreduktion-Eingabemittel einen Knopf, einen Schalter, oder einen Regler, der von Hand betätigt werden kann. Der Bediener kann somit das Kraftreduktion-Eingabemittel, während er beispielsweise auf dem Behandlungstisch liegt, in seiner Hand halten und die Anpresskraftreduktion direkt mit seiner Hand steuern. Wenn der Manipulator eine Kraft auf den Patienten ausübt, kann der Patient durch Drücken des Knopfs diese Kraft intuitiv reduzieren. Alternativ oder zusätzlich können auch andere Benutzer-Schnittstellen verwendet werden, wie beispielsweise andere Knopfarten, Stellvorrichtungen, Fußschalter, Sprachschnittstellen, eine Brain-Maschine-Interface, Gesichtserkennung, Emotionserkennung, Augentracking, Patientenposen-Tracking (z.B. mittels simpler Laserschranken), etc.

Vorzugsweise umfasst der Manipulator Kraft- und/oder Momentsensoren, welche beispielsweise mittels Dehnungsmessestreifen die auf den Manipulator wirkenden Kräfte bzw. Drehmomente erfassen können. Alternativ können auch Motorströme ausgewertet werden, welche in den Antrieben des Manipulators auftreten. Vorzugsweise ist das Steuermittel dabei derart eingerichtet, den Manipulator mittels Kraft-, Positions- oder Hybridregelung anzusteuern, wobei die Hybridregelung eine Mischform von Kraft- und Positionsregelung bezeichnet. Die Reduktion der Anpresskraft kann sich dabei vorzugsweise direkt auf diese Führungsgröße auswirken. So kann bei einer reinen Positionsregelung die Reduktion der Kraft direkt über die Regelung der Position als Führungsgröße geschehen. Durch den Einsatz von Kraft-und/oder Momentsensoren kann sehr effizient die Anpresskraft direkt ermittelt werden, und gemäß der Eingabe des Bedieners reduziert werden.

Vorzugsweise ist das Kraftreduktion-Eingabemittel ferner von einem Bediener zum Abbrechen des Anpressens betätigbar. Das Kraftreduktion-Eingabemittel umfasst somit eine zusätzliche Funktion, welche es einem Bediener erlaubt, den Einsatz bzw. die aktuelle Behandlung abzubrechen. Dazu ist das Steuermittel vorzugsweise ferner eingerichtet den Manipulator derart anzusteuern, dass die Anpresskraft auf Null reduziert wird und dass das Instrument von Körper entfernt wird oder manuell wegbewegbar wird (indem der Manipulator bspw. in einen "weichen" Impedanzmodus (auch Handführmodus bezeichnet) geschaltet wird, in dem er sich durch den Patienten wegdrücken lässt), basierend auf einer entsprechenden Betätigung des Kraftreduktion-Eingabemittels. Durch die Aufforderung durch den Bediener, dass Anpressen abzubrechen, kann somit eine Art "Panikmodus" aktiviert werden. In diesem Fall wird nicht lediglich die Anpresskraft reduziert, sondern der Berührungskontakt abgebrochen und das Verfahren vorzugsweise abgebrochen. Somit erhält der Bediener zusätzlich zu der Möglichkeit der Anpresskraftreduktion auch die Möglichkeit, das Verfahren abzubrechen, sollte ein notwendiger Vertrauensgewinn durch die Anpresskraftreduktion nicht eintreten.

Vorzugsweise ist das Kraftreduktions-Eingabemittel analog betätigbar. Das Kraftreduktion-Eingabemittel kann beispielsweise kontinuierlich zwischen zwei Endpositionen betätigbar sein. Das Kraftreduktion-Eingabemittel kann somit vorzugsweise als analoger Regler ausgestaltet sein. Das Steuermittel ist dabei vorzugsweise eingerichtet den Manipulator derart anzusteuern, dass die Anpresskraft abhängig von einem Ausmaß der entsprechenden analogen Betätigung des Kraftreduktion-Eingabemittels reduziert wird. Die Reduktion der Kraft kann somit abhängig von der Drucktiefe des analogen Reglers sein. Somit kann der Bediener präzise vorgeben, in welchem Maße die Anpresskraft reduziert werden soll. Insbesondere vorzugsweise ist das Steuermittel eingerichtet den Manipulator derart anzusteuern, dass die Anpresskraft proportional oder nicht-linear zum Ausmaß der entsprechenden analogen Betätigung des Kraftreduktion-Eingabemittels reduziert wird, oder das die Anpresskraft basierend auf einer zeitlichen Ableitung des Ausmaßes der entsprechenden analogen Betätigung des Kraftreduktion-Eingabemittels reduziert wird. Die Drucktiefe steht somit vorzugsweise in einem direkten Verhältnis zur resultierenden Anpresskraftreduktion. In einem bevorzugten Ausführungsbeispiel kann je nach Geschwindigkeit bzw. Beschleunigung, mit welcher die analoge Betätigung erfolgt, das Ausmaß der Kraftreduktion beeinflusst werden.

Vorzugsweise ist das Kraftreduktion-Eingabemittel digital betätigbar. Dabei kann das Kraftreduktion-Eingabemittel nicht kontinuierlich, sondern diskret betätigbar sein. Beispielsweise kann ein digitaler Schalter verwendet werden, mittels welchem die aufgebrachte Anpresskraft des Manipulators reduziert werden kann, oder direkt in einen "Panik-Modus" geschaltet werden kann. Dabei ist das Steuermittel vorzugsweise eingerichtet den Manipulator derart anzusteuern, dass die Anpresskraft kontinuierlich während einer anhaltenden entsprechenden digitalen Betätigung und/oder in Einzelschritten während einer wiederholten entsprechenden digitalen Betätigung und/oder um einen vordefinierten Faktor bzw. auf einen vordefinierten Betrag während einer einzelnen entsprechenden digitalen Betätigung reduziert wird. Beispielsweise kann, wenn ein digitaler Knopf verwendet wird, die Anpresskraft solange kontinuierlich weiter reduziert werden, wie der Knopf gedrückt bleibt, und dann die Kraft aufrechterhalten bleibt, die beim Loslassen des Knopfes anliegt. Der digitale Knopf könnte auch durch wiederholtes Drücken die Kraft in Einzelschritten reduzieren. Durch einmaliges Drücken des digitalen Knopfs könnte auch die Kraft beispielsweise um 50% reduziert werden, und anschließend langsam wieder erhöht werden, bis der Knopf beispielsweise ein weiteres Mal gedrückt wird. Somit können sehr individuelle Reaktionsstrategien realisiert werden.

Vorzugsweise ist das Kraftreduktion-Eingabemittel eingerichtet, eine haptische Rückmeldung, vorzugsweise mittels Vibration, an den Bediener auszugeben. So kann beispielsweise mittels Vibrationsstärke oder Vibrationsintervallen eines Handgriffs des Kraftreduktion-Eingabemittels dem Bediener bzw. Patienten eine Information über die Behandlung bzw. Untersuchung mitgeteilt werden. Beispielsweise kann mittels einer Vibration der Anfang oder das Ende eines Untersuchungsabschnitts angekündigt werden.

Vorzugsweise ist das Steuermittel ferner eingerichtet, den Manipulator derart anzusteuern, dass eine Bewegung des Instruments entlang des Körpers unterbrochen wird und/oder das Instrument vom Körper entfernt wird, wenn durch das Manipulatorsystem ermittelt wird, dass die auf einen Betrag bzw. Wert größer Null reduzierte Anpresskraft einen Anpresskraft-Grenzwert untersteigt bzw. unterschreitet. Wenn beispielsweise die reduzierte Anpresskraft zu niedrig ist, um das gewünschte Untersuchungs- bzw. Behandlungsergebnis zu erzielen, könnte das System vorteilhaft die Applikation unterbrechen und eine entsprechende Ausgabe an einen Arzt generieren, der die Untersuchung bzw. Behandlung betreut. Diese Person könnte dann beruhigend oder aufklärend auf den Patienten einwirken oder auch entscheiden, auf eine alternative manuelle Untersuchung bzw. Behandlung zu wechseln. Alternativ könnte die Unterbrechung auch derart ausgestaltet sein, dass falls der Manipulator über Kraft- und/oder Momentsensoren verfügt und bspw. mittels Impedanzregelung (also einer besonderen Unterform der Kraftregelung) betrieben wird, der Manipulator die letzte Pose hält und die Anpresskraft weiterhin vom Patienten kontrolliert wird. Wenn der Patient wieder eine erhöhte Kraft zulässt, durch entsprechende Betätigung des Kraftreduktion-Eingabemittels, kann die Bahnplanung fortgesetzt werden. Alternativ könnte die Untersuchung bzw. Behandlung auch mit der zu niedrigen Anpresskraft fortgesetzt werden, und ggf. zu einem späteren Zeitpunkt eine gezielte zweite Untersuchung bzw. Behandlung durchgeführt werden. Die Stellung eines analogen Handreglers, welche den Anpresskraft-Grenzwert kennzeichnet, könnte vorteilhaft auch haptisch über das Kraftreduktion-Eingabemittel signalisiert werden, zum Beispiel durch einen spürbaren Wiederstand beim Überschreiten dieser Schwelle.

Vorzugsweise ist das Messwert-Auswertemittel ferner eingerichtet festzustellen, ob die erfasste Qualität der Messwerte einen Qualität-Grenzwert unterschreitet. Insbesondere vorzugsweise ist das Steuermittel ferner eingerichtet den Manipulator derart anzusteuern, dass eine Bewegung des Instruments entlang des Körpers unterbrochen und/oder das Instrument vom Körper entfernt wird, wenn durch das Messwert-Auswertemittel festgestellt wird, dass die erfasste Qualität der Messwerte den Qualität-Grenzwert unterschreitet. Wenn also die reduzierte Anpresskraft einen entsprechend hohen Qualitätsverlust der Messwerte mit sich bringt, kann beispielsweise die Behandlung bzw. Untersuchung pausiert oder auch abgebrochen werden, wie oben beschrieben.

Vorzugsweise umfasst das Manipulatorsystem weiterhin Sensoren zur Überwachung von Vitalzeichen des menschlichen Körpers zur Ermittlung eines Stresszustandes. Beispielsweise kann durch Messen des Pulses oder der Körpertemperatur auf einen Angst- bzw. Stresszustand des Patienten geschlossen werden. Ferner umfasst das Manipulatorsystem vorzugsweise ein Stress-Auswertemittel, welches eingerichtet ist, anhand der überwachten Vitalzeichen einen Stresszustand des Körpers zu erfassen und festzustellen, ob der erfasste Stresszustand einen Stressgrenzwert übersteigt. Dabei ist das Steuermittel vorzugsweise ferner eingerichtet den Manipulator derart anzusteuern, dass die Anpresskraft auf Null reduziert wird und das Instrument vom Körper entfernt wird oder manuell entfernt werden kann, wenn durch das Stress-Auswertemittel festgestellt wird, dass der erfasste Stresszustand den Stressgrenzwert übersteigt. Somit kann unabhängig von dem Kraftreduktion-Eingabemittel der "Panikmodus" aktiviert werden, wenn ein entsprechender Panikzustand des Patienten erkannt wird. Somit wird verhindert, dass wenn der Patient bspw. in einer Schockstarre unfähig ist das Kraftreduktion-Eingabemittel zu bedienen, trotzdem der Panikmodus eingeleitet wird.

Vorzugsweise ist das Steuermittel ferner eingerichtet den Manipulator derart anzusteuern, dass die Anpresskraft stetig erhöht wird, und wobei das Manipulatorsystem vorzugsweise eingerichtet ist, eine maximale zulässige Anpresskraft zu bestimmen, basierend auf einer erstmaligen (während die Anpresskraft stetig erhöht wird) Betätigung des Kraftreduktion-Eingabemittels. Somit kann vorteilhaft das persönliche Schmerzempfinden des Patienten erfasst werden. In einem vor der eigentlichen Untersuchung stattfindenden Initialisierungsschritt kann somit das Instrument beispielsweise mit stetig zunehmender Anpresskraft auf eine Hand des Patienten gepresst werden, bis der Patient durch eine entsprechende Betätigung des Kraftreduktion-Eingabemittels signalisiert, dass sein persönliches Schmerzempfinden erreicht wurde. Die entsprechende Anpresskraft kann in dem System als maximal zulässige Anpresskraft gespeichert und für die folgenden Behandlungsschritte verwendet werden.

Durch eine Betätigung des Kraftreduktion-Eingabemittels zur Reduktion der Anpresskraft wird die Anpresskraft bzw. der Druck nicht zwingend mechanisch aufgehoben. Die Anwendung wird somit nicht notwendigerweise beendet. Vielmehr kann die Untersuchung mit verringertem Druck fortgeführt werden oder gegebenenfalls pausiert werden. Vorzugsweise wird mittels des Eingabemittels kein direkter Einfluss auf die Mechanik des Manipulators genommen. Vielmehr wird die Betätigung über die elektronische Manipulatorsteuerung ausgewertet und anschließend in eine entsprechende Steuerung umgesetzt.

Die Reduktion der Anpresskraft kann mittels einer vorgegebenen mathematischen Funktion erfolgen, die als Eingangsvariablen die Dauer der Betätigung berücksichtigt. Als andere oder zusätzliche Eingangsvariable kann auch die Höhe des aktuell vom Manipulator erzeugten Drucks bzw. Anpresskraft verwendet werden, sodass bei einer sehr hohen Anpresskraft die Reduktion schneller erfolgt, als wenn die Anpresskraft bereits gering ist.

Die Erfindung betrifft weiterhin ein Verfahren zum Steuern eines Manipulators, wobei der Manipulator ein Instrument führt. Das Verfahren umfasst dabei ein Pressen des Instruments gegen einen menschlichen Körper mit einer Anpresskraft. Dieses Pressen kann im Rahmen des Abfahrens einer Bahnplanung erfolgen.

In einem weiteren Schritt wird, während des Pressens, detektiert, ob eine Betätigung an einem Kraftreduktion-Eingabemittel vorliegt, wobei das Eingabemittel separat zum Manipulator bereitgestellt ist und von einem Bediener betätigbar ist zur Reduktion der Anpresskraft Außerdem werden Messwerte mittels des Instruments erfasst.

In einem weiteren Schritt wird die Qualität der Messwerte mit einem Messwert-Auswertemittel erfasst, wobei das Messwert-Auswertemittel separat zum Manipulator bereitgesellt wird.

In einem weiteren Schritt wird der Manipulator basierend auf der erfassten Qualität der Messwerte angesteuert.

Ferner wird, wenn detektiert wird, dass eine entsprechende Betätigung des Kraftreduktion-Eingabemittels vorliegt, der Manipulator angesteuert um die Anpresskraft auf einen Betrag bzw. Wert größer Null zu reduzieren.

Der Fachmann versteht, dass die oben in Bezug auf das Manipulatorsystem beschriebenen Komponenten auch entsprechende Schritte in einem erfindungsgemäßen Verfahren darstellen bzw. beschreiben können. So kann beispielsweise das Eingabemittel betätigbar sein zum Abbruch der Anpresskraft, und wenn detektiert wird, dass eine entsprechende Betätigung des Kraftreduktion-Eingabemittels vorliegt, der Manipulator angesteuert werden um die Anpresskraft auf Null zu reduzieren und das Instrument vom Körper zu entfernen. Ferner kann vorzugsweise in einem Initialisierungsschritt die Anpresskraft stetig erhöht werden, bis das Eingabemittel bei einer maximal zulässigen Anpresskraft erstmalig betätigt wird. Ferner kann die Behandlung abgebrochen werden, wenn detektiert wird, dass die reduzierte Anpresskraft einen Anpresskraftgrenzwert unterschreitet. Ferner kann, wenn eine erfasste Qualität der Messwerte einen Qualität-Grenzwert unterschreitet, die Behandlung abgebrochen werden. Auch kann, analog zu der obigen Beschreibung, zumindest ein Vitalzeichen des menschlichen Körpers überwacht werden und basierend hierauf ein Stresszustand des Körpers ermittelt werden, und die Anpresskraft auf Null reduziert werden und das Instrument vom Körper entfernt werden, wenn detektiert wird, dass der ermittelte Stresszustand den Stress-Grenzwert übersteigt. Diese Aufzählung bevorzugter Verfahrensschritte ist nicht abschließend, sondern ergänzt sich für den Fachmann um die oben hinsichtlich des Manipulatorsystem beschriebenen Details.

Gemäß der Erfindung kann ein Manipulatorsystem, etwa wie oben beschrieben, zur Verwendung bei der Behandlung eines Patienten eingesetzt werden, wobei die Verwendung zumindest teilweise gemäß der obigen Beschreibung erfolgen kann.

### 4. Beschreibung bevorzugter Ausführungsbeispiele

Im Folgenden wird die vorliegende Erfindung unter Bezugnahme auf die beiliegenden Figuren näher beschrieben. Dabei zeigt:
Figur 1 ein Manipulatorsystem gemäß einer Ausführungsform der vorliegenden Erfindung, und
Figur 2 ein Kraftreduktion-Eingabemittel gemäß einer Ausführungsform der vorliegenden Erfindung.

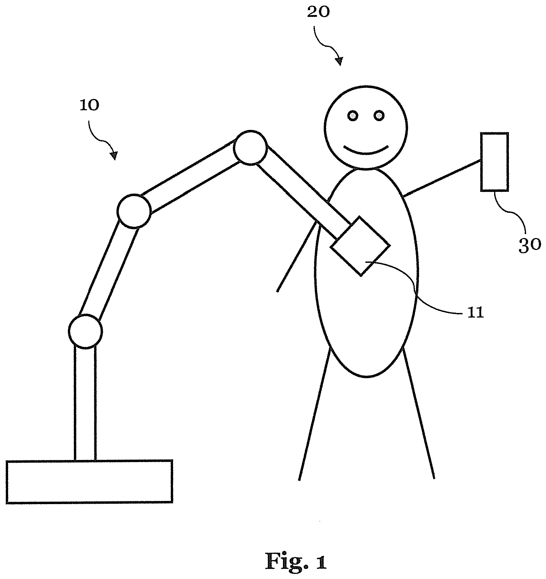

In der Figur 1 ist ein Manipulatorsystem dargestellt, mittels welchem eine Behandlung an einem menschlichen Körper bzw. Patienten 20 durchgeführt werden kann. Dazu ist ein Manipulator 10 bereitgestellt, welcher als mehrachsiger Gelenkarmroboter ausgestaltet ist. Der Manipulator 10 weist als Endeffektor ein Instrument 11 auf, welches beispielsweise als Ultraschallmesskopf 11 ausgestaltet ist. Die Achsen des Manipulators 10 sind mit Kraft-Moment-Sensoren ausgestattet, welche es erlauben, Kräfte bzw. Momente zu erfassen, die auf den Manipulator 10 wirken. Somit kann eine Anpresskraft detektiert werden, die von dem Manipulator 10 auf den Körper 20 ausgeübt wird. Der Manipulator 10 wird dabei mittels eine hybriden Regelung gesteuert, welche eine Kombination aus Impedanz- und Positionsregelung ist.

Der Patient 20 hält in seiner Hand ein Kraftreduktion-Eingabemittel 30, welches in Form einer Kraftreduktion-Eingabevorrichtung 30 ausgestaltet ist. Diese ist kabellos mit dem Manipulator 10 gekoppelt, beispielsweise mittels Funk- oder Lichtwellentechnologie. Durch entsprechendes Betätigen der Kraftreduktion-Eingabevorrichtung 30 kann der Patient 20 die Anpresskraft, die von dem Manipulator 10 mittels des Instruments 11 auf den Patienten 20 ausgeübt wird, reduzieren.

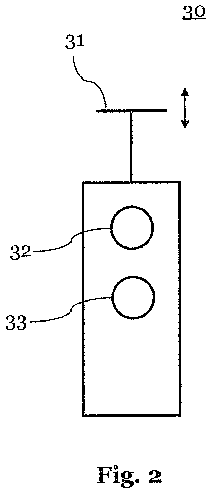

In der Figur 2 ist eine Ausführungsform des erfindungsgemäßen Kraftreduktion-Eingabemittels 30 dargestellt, wie etwa in der Situation der Figur 1 verwendet. Das Kraftreduktion-Eingabemittel ist als Eingabevorrichtung 30 bzw. als Handgerät 30 ausgestaltet, welches von dem Patienten 20 in der Hand gehalten und per Hand betätigt werden kann. Die Kraftreduktion-Eingabevorrichtung 30 umfasst dabei einen analogen Regler 31, und zwei digitale Knöpfe 32, 33.

Durch entsprechende Betätigung des analogen Reglers 31 kann der Patient 20 präzise vorgeben, wie die Kraftreduktion erfolgen soll. Je nachdem, wie weit der analoge Regler 31 gedrückt wird, wird das Maß der Reduktion der Anpresskraft gesteuert. Auch durch die Geschwindigkeit des Drückens des analogen Reglers 31 kann bestimmt werden, wie schnell oder in welchem Maße die Kraftreduktion erfolgen soll. Vorteilhaft kann durch ein volles Durchdrücken des analogen Reglers 31 ein "Panikmodus" eingeleitet werden, bei dem die Applikation beendet wird und der Manipulator 10 das Instrument 11 von dem Patienten 20 entfernt. Diese Stellung des analogen Reglers 31 kann durch einen erhöhten haptischen Widerstand gekennzeichnet werden, damit ein versehentliches Wechseln in den "Panikmodus" verhindert wird. Bei Eintritt in den "Panikmodus" kann die Anpresskraft auf Null reduziert werden und sich der Manipulator 10 langsam vom Patienten 20 entfernen. Dazu oder alternativ kann der Manipulator, aufgrund seiner Kraft-und-Momentsensorik, auch in einen "weichen" Impedanzmodus geschaltet werden, sodass er sich problemlos durch den Patienten 20 wegdrücken lässt.

Der "Panikmodus" kann auch durch einen weiteren Knopf bzw. Sensor im Handgriff 30 ausgelöst werden. Beispielsweise kann der Knopf 32 als ein solcher Panik-Knopf verwendet werden. Der Knopf 32 kann ähnlich zu einem Zustimmschalter funktionieren, der leicht gedrückt werden muss, damit der Manipulator 10 seinen Bewegungsablauf durchführen kann. Wenn der Knopf 32 vollständig oder gar nicht gedrückt wird, kann dies als Auslöser für den "Panikmodus" gewertet werden.

Der Knopf 33 kann als digitaler Schalter als Benutzerschnittstelle eingesetzt werden, um die Anpresskraftreduktion vorzugeben. Während des Drückens des Knopfes 33 wird die Anpresskraft kontinuierlich reduziert, bis der Knopf 33 losgelassen wird. Durch wiederholtes, schnelles Drücken des Knopfes 33 kann ebenfalls der "Panikmodus" aktiviert bzw. ausgelöst werden.

Der Fachmann versteht, dass ein Kraftreduktion-Eingabemittel gemäß der vorliegenden Erfindung ein oder mehrere der beschriebenen Regler bzw. Knöpfe 31, 32, 33 aufweisen kann. Zumindest muss ein Kraftreduktion-Eingabemittel gemäß der vorliegenden Erfindung das Erkennen einer Eingabe des Bedieners ermöglichen, welche eine Anpresskraftreduktion vorgibt. Zusätzliche Regler bzw. Knöpfe können für zusätzliche Funktionen, wie beispielsweise zum Auslösen des "Panikmodus" eingesetzt werden.

Im Folgenden wird ein Ausführungsbeispiel der vorliegenden Erfindung beschrieben, wobei exemplarisch auf die Komponenten der Figur 1 bzw. der Figur 2 Bezug genommen wird.

Der Manipulator 10 wird in dem vorliegenden Ausführungsbeispiel eingesetzt, um den Ultraschallkopf 11 über den Bauch des Patienten 20 zu führen, sodass die Aorta des Patienten 20 untersucht werden kann. Die Kräfte, die hierbei auf den Patienten aufgebracht werden müssen, variieren mit der Anatomie des Patienten. Der Fachmann versteht, dass hierbei Kräfte von oberhalb 20 N nicht unüblich sind.

Vor der eigentlichen Untersuchung wird das System in einen Demonstrations-Modus versetzt. Hierbei hält der Patient 20 die Eingabevorrichtung 30 in einer Hand, während auf die geöffnete Handfläche der anderen Hand der manipulatorgeführte Ultraschallkopf 11 aufgelegt wird. Der Manipulator 10 erzeugt nun eine Kraft gegen die Handfläche und der Patient 20 kann ausprobieren, wie er diese Anpresskraft mithilfe der Eingabevorrichtung 30 reduzieren kann. Dieser Demonstrations-Modus dient somit der Schaffung von Vertrauen des Patienten 20 in das System.

In einem Initialisierungsschritt kann nun die maximal zulässige Anpresskraft gemessen werden: Hierzu presst der Manipulator 10 den Ultraschallkopf 11 wieder gegen die Handfläche (oder eine andere Stelle, wie vorzugsweise an einer Stelle, an der die eigentliche Untersuchung stattfinden soll) des Patienten 20, wobei die Anpresskraft stetig erhöht wird. Sobald die persönliche Schmerzgrenze des Patienten 20 erreicht wird, betätigt dieser die Eingabevorrichtung 30. Die momentan vorliegende Anpresskraft wird als maximal zulässige Anpresskraft im System hinterlegt und das Instrument 11 wieder entfernt.

Nun wird der Patient 20 für die Untersuchung präpariert und gelagert, wobei er weiterhin die Eingabevorrichtung 30 in seiner Hand hält. Der Manipulator 10 fährt daraufhin die geplante Trajektorie für die Aorta-Untersuchung mittels Impedanzregelung ab. Die ersten Schritte dienen hier üblicherweise einem Kalibrierungsschritt, bei welchem der Manipulator 10 zunächst die optimale Orientierung des Ultraschallkopfes 11 und die notwendige Kraft bestimmt, bevor er die tatsächliche Untersuchung entlang der Aorta-Lage vollzieht. Zu jeder Zeit kann der Patient 20 diese optimale Kraft reduzieren, indem er die Eingabevorrichtung 30 betätigt. Dabei wird die Anpresskraft abhängig von der Betätigung der Eingabevorrichtung 30 bis zu einer festgelegten bzw. vordefinierten Minimal-Kraft verringert.

Sowohl für den Demonstrationsmodus als auch für den normalen Untersuchungsablauf kann die Impedanzregelung des Manipulators 10 derart konfiguriert sein, dass eine in Stoßrichtung des Ultraschallkopfes 11 erzeugte Kraft multipliziert wird mit einem entsprechend der Betätigung der Eingabevorrichtung 30 erzeugten Faktor, um die Anpresskraft zu reduzieren. Ein voll durchgedrückter Regler 31 könnte beispielsweise einem Faktor von 0,01 entsprechen, und ein nicht betätigter Regler 31 dem Faktor 1. In alle anderen Richtungen sowie in der Orientierung ist die Impedanzregelung mit hoher Steifigkeit eingestellt, um Bewegungen in diesen Freiheitsgraden zu verhindern. Beim Einsatz einer hybriden Regelung könnten diese Steifheitsgrade dann auch positionsgeregelt sein.

Vorzugsweise kann auch eine mittels des Manipulators 10 erfolgte Messung der Anpresskraft berücksichtigt werden zur Reduktion dieser Anpresskraft. Dies wird durch die Kraft-Moment-Sensorik des Manipulators 10 ermöglicht. Vorzugsweise wird die Kraftreduktion lediglich durch eine Bewegung des Manipulatorarms herbeigeführt, sodass Kollisionen des Manipulators 10 mit der Umgebung verhindert werden.

Vorzugsweise kann, da der Manipulator 10 mit Kraft-Moment-Sensorik ausgestattet ist, eine Abweichung von erwarteten applikationstypischen Kräften erfasst werden und auch hier beim Überschreiten einer erlaubten Toleranzschwelle der beschriebene "Panikmodus" ausgelöst werden. Solche Abweichungen könnten beispielsweise die Kraftrichtung, Krafthöhe oder den Angriffspunkt einer Kraft betreffen. Somit kann detektiert werden, dass der Patient 20 versucht den Manipulator 10 wegzudrücken. Der Patient 20 könnte beispielsweise derart verunsichert sein, dass er die Eingabevorrichtung 30 nicht beachtet, sondern instinktiv versucht den Manipulator 10 wegzudrücken.

Obwohl in der vorliegenden Beschreibung beschrieben wurde, dass der Patient die Eingabevorrichtung bedient, kann auch eine betreuende Person bzw. Arzt die Eingabevorrichtung in der Hand halten und die Kraft anhand von Rücksprache mit dem Patienten reduzieren. Die beste Wirkung auf die Vertrauensbildung zum System wird jedoch erzielt, wenn der Patient selbst die Reduktion der Anpresskraft vorgibt.

## Patentansprüche

1. Manipulatorsystem, aufweisend:
- einen Manipulator (10) eingerichtet zum Führen eines Instruments (11);
- ein Steuermittel, welches eingerichtet ist, den Manipulator (10) derart anzusteuern, dass das Instrument (11) mit einer Anpresskraft gegen einen menschlichen Körper (20) gepresst wird;
- ein Kraftreduktion-Eingabemittel (30), welches separat zum Manipulator (10) bereitgestellt ist und von einem Bediener (20) betätigbar ist zur Reduktion der Anpresskraft;
- wobei das Steuermittel ferner eingerichtet ist den Manipulator (10) derart anzusteuern, dass die Anpresskraft auf einen Betrag größer Null reduziert wird, basierend auf einer entsprechenden Betätigung des Kraftreduktion-Eingabemittels (30), **dadurch gekennzeichnet, dass**
- das Manipulatorsystem eingerichtet ist, während des Pressens Messwerte mittels des Instruments (11) zu erfassen; dass
- das Manipulatorsystem ferner ein Messwert-Auswertemittel aufweist, welches eingerichtet ist, eine Qualität der Messwerte zu erfassen; und dass
- das Steuermittel ferner eingerichtet ist den Manipulator (10) basierend auf der erfassten Qualität der Messwerte anzusteuern.

2. Manipulatorsystem nach Anspruch 1, wobei das Kraftreduktion-Eingabemittel (30) eine Benutzer-Schnittstelle ist, welche einen Knopf, Schalter oder Regler umfasst, welcher vorzugsweise per Hand betätigt werden kann.

3. Manipulatorsystem nach einem der Ansprüche 1 bis 2, wobei der Manipulator (10) Kraft- und/oder Momentsensoren umfasst, und wobei das Steuermittel vorzugsweise eingerichtet ist den Manipulator (10) mittels Kraft-, Positions- oder Hybridregelung anzusteuern.

4. Manipulatorsystem nach einem der Ansprüche 1 bis 3,
- wobei das Kraftreduktion-Eingabemittel (30) ferner von einem Bediener (20) betätigbar ist zum Abbrechen des Anpressens;
- wobei das Steuermittel ferner eingerichtet ist den Manipulator (10) derart anzusteuern, dass die Anpresskraft auf Null reduziert wird und das Instrument (11) vom Körper (20) entfernt wird oder manuell entfernt werden kann, basierend auf einer entsprechenden Betätigung des Kraftreduktion-Eingabemittels (30).

5. Manipulatorsystem nach einem der Ansprüche 1 bis 4,
- wobei das Kraftreduktion-Eingabemittel (30) analog betätigbar ist;
- wobei das Steuermittel eingerichtet ist den Manipulator (10) derart anzusteuern, dass die Anpresskraft abhängig von einem Ausmaß der entsprechenden analogen Betätigung des Kraftreduktion-Eingabemittels (30) reduziert wird.

6. Manipulatorsystem nach Anspruch 5, wobei das Steuermittel eingerichtet ist den Manipulator (10) derart anzusteuern, dass die Anpresskraft proportional oder nicht-linear zum Ausmaß der entsprechenden analogen Betätigung des Kraftreduktion-Eingabemittels (30) oder basierend auf einer zeitlichen Ableitung des Ausmaßes der entsprechenden analogen Betätigung des Kraftreduktion-Eingabemittels (30) reduziert wird.

7. Manipulatorsystem nach einem der Ansprüche 1 bis 6,
wobei das Kraftreduktion-Eingabemittel (30) digital betätigbar ist;
- wobei das Steuermittel eingerichtet ist den Manipulator (10) derart anzusteuern, dass die Anpresskraft kontinuierlich während einer anhaltenden entsprechenden digitalen Betätigung und/oder in Einzelschritten während einer wiederholten entsprechenden digitalen Betätigung und/oder um einen vordefinierten Faktor bzw. auf einen vordefinierten Wert während einer einzelnen entsprechenden digitalen Betätigung reduziert wird.

8. Manipulatorsystem nach einem der Ansprüche 1 bis 7, wobei das Kraftreduktion-Eingabemittel (30) eingerichtet ist, eine haptische Rückmeldung vorzugsweise mittels Vibration an den Bediener (20) auszugeben.

9. Manipulatorsystem nach einem der Ansprüche 1 bis 8, wobei das Steuermittel ferner eingerichtet ist den Manipulator (10) derart anzusteuern, dass eine Bewegung des Instruments (11) entlang des Körpers (20) unterbrochen wird und/oder das Instrument (11) vom Körper (20) entfernt wird, wenn durch das Manipulatorsystem ermittelt wird, dass die auf einen Betrag größer Null reduzierte Anpresskraft einen Anpresskraft-Grenzwert untersteigt.

10. Manipulatorsystem nach Anspruch 1,
- wobei das Messwert-Auswertemittel ferner eingerichtet ist festzustellen, ob die erfasste Qualität der Messwerte einen Qualität-Grenzwert unterschreitet;
- wobei das Steuermittel ferner eingerichtet ist den Manipulator (10) derart anzusteuern, dass eine Bewegung des Instruments (11) entlang des Körpers (20) unterbrochen und/oder das Instrument (11) vom Körper (20) entfernt wird, wenn durch das Messwert-Auswertemittel festgestellt wird, dass die erfasste Qualität der Messwerte den Qualität-Grenzwert unterschreitet.

11. Manipulatorsystem nach einem der Ansprüche 1 bis 10, weiter aufweisend:
Sensoren zur Überwachung von Vitalzeichen des Körpers (20) zur Ermittlung eines Stresszustandes;
- ein Stress-Auswertemittel welches eingerichtet ist, anhand der überwachten Vitalzeichen einen Stresszustand des Körpers (20) zu erfassen und festzustellen, ob der erfasste Stresszustand einen Stress-Grenzwert übersteigt;
- wobei das Steuermittel ferner eingerichtet ist den Manipulator (10) derart anzusteuern, dass die Anpresskraft auf Null reduziert wird und das Instrument (11) vom Körper (20) entfernt wird oder manuell entfernt werden kann, wenn durch das Stress-Auswertemittel festgestellt wird, dass der erfasste Stresszustand den Stress-Grenzwert übersteigt.

12. Manipulatorsystem nach einem der Ansprüche 1 bis 11, wobei das Steuermittel ferner eingerichtet ist den Manipulator (10) derart anzusteuern, dass die Anpresskraft stetig erhöht wird, und wobei das Manipulatorsystem eingerichtet ist eine maximal zulässige Anpresskraft zu bestimmen basierend auf einer erstmaligen Betätigung des Kraftreduktion-Eingabemittels (30), und zwar während die Anpresskraft stetig erhöht wird.

13. Verfahren zum Steuern eines Manipulators (10), wobei der Manipulator (10) ein Instrument (11) führt, aufweisend:
- Pressen des Instruments (11) gegen einen menschlichen Körper (20) mit einer Anpresskraft;
- Während des Pressens: Detektieren, ob eine Betätigung an einem Kraftreduktion-Eingabemittel (30) vorliegt, wobei das Eingabemittel (30) separat zum Manipulator (10) bereitgestellt ist und von einem Bediener (20) betätigbar ist zur Reduktion der Anpresskraft und Erfassen von Messwerten mittels des Instruments (11);
- Erfassen einer Qualität der Messwerte mit einem Messwert-Auswertemittel, wobei das Messwert-Auswertemittel separat zum Manipulator (10) bereitgestellt ist;
- Ansteuern des Manipulators (10) basierend auf der erfassten Qualität der Messwerte und wenn detektiert wird, dass eine entsprechende Betätigung des Kraftreduktion-Eingabemittel (30) vorliegt: Ansteuern des Manipulators (10) um die Anpresskraft auf einen Betrag größer Null zu reduzieren.

14. Manipulatorsystem nach einem der Ansprüche 1 bis 12 oder Verfahren nach Anspruch 13, wobei das Instrument (11) zur Bildgebung und insbesondere zur Tomografie eingerichtet ist.

## Claims

1. Manipulator system comprising:
- a manipulator (10) configured to guide an instrument (11);
- a control means which is configured to control the manipulator (10) in such a way that the instrument (11) is pressed against a human body (20) with a pressing force;
- a force reduction input means (30) which is provided separately from the manipulator (10) and is actuatable by an operator (20) for reduction of the pressing force;
- wherein the control means is also configured to control the manipulator (10) in such a way that the pressing force is reduced to an amount greater than zero, based on a corresponding actuation of the force reduction input means (30), **characterized in that**
- the manipulator system is configured to acquire measured values by means of the instrument (11) during the pressing; **in that**
- the manipulator system further comprises a measured value evaluation means which is configured to acquire a quality of the measured values; and **in that**
- the control means is also configured to control the manipulator (10) based on the acquired quality of the measured values.

2. Manipulator system according to Claim 1, wherein the force reduction input means (30) is a user interface which comprises a button, switch or controller which can preferably be actuated by hand.

3. Manipulator system according to one of Claims 1 to 2, wherein the manipulator (10) comprises force and/or torque sensors, and wherein the control means is preferably configured to control the manipulator (10) by means of force, position or hybrid control.

4. Manipulator system according to one of Claims 1 to 3,
- wherein the force reduction input means (30) is also actuatable by an operator (20) for termination of the pressing;
- wherein the control means is also configured to control the manipulator (10) in such a way that the pressing force is reduced to zero and the instrument (11) is removed or can be manually removed from the body (20), based on a corresponding actuation of the force reduction input means (30).

5. Manipulator system according to one of Claims 1 to 4,
- wherein the force reduction input means (30) is actuatable in an analogue manner;
- wherein the control means is configured to control the manipulator (10) in such a way that the pressing force is reduced depending on an extent of the corresponding analogue actuation of the force reduction input means (30).

6. Manipulator system according to Claim 5, wherein the control means is configured to control the manipulator (10) in such a way that the pressing force is reduced in a proportional or non-linear manner with respect to the extent of the corresponding analogue actuation of the force reduction input means (30) or based on a time derivative of the extent of the corresponding analogue actuation of the force reduction input means (30).

7. Manipulator system according to one of Claims 1 to 6,
- wherein the force reduction input means (30) is actuatable in a digital manner;
- wherein the control means is configured to control the manipulator (10) in such a way that the pressing force is reduced continuously during a sustained corresponding digital actuation and/or in individual steps during a repeated corresponding digital actuation and/or by a predefined factor or to a predefined value during an individual corresponding digital actuation.

8. Manipulator system according to one of Claims 1 to 7, wherein the force reduction input means (30) is configured to output haptic feedback to the operator (20) preferably by means of vibration.

9. Manipulator system according to one of Claims 1 to 8, wherein the control means is also configured to control the manipulator (10) in such a way that a movement of the instrument (11) along the body (20) is interrupted and/or the instrument (11) is removed from the body (20) if the manipulator system ascertains that the pressing force reduced to an amount greater than zero is below a pressing force limit value.

10. Manipulator system according to Claim 1,
- wherein the measured value evaluation means is also configured to determine whether the acquired quality of the measured values is below a quality limit value;
- wherein the control means is also configured to control the manipulator (10) in such a way that a movement of the instrument (11) along the body (20) is interrupted and/or the instrument (11) is removed from the body (20) if the measured value evaluation means determines that the acquired quality of the measured values is below the quality limit value.

11. Manipulator system according to one of Claims 1 to 10, further comprising:
- sensors for monitoring vital signs of the body (20) for ascertainment of a stress level;
- a stress evaluation means which is configured to acquire a stress level of the body (20) on the basis of the monitored vital signs and to determine whether the acquired stress level exceeds a stress limit value;
- wherein the control means is also configured to control the manipulator (10) in such a way that the pressing force is reduced to zero and the instrument (11) is removed or can be manually removed from the body (20) if the stress evaluation means determines that the acquired stress level exceeds the stress limit value.

12. Manipulator system according to one of Claims 1 to 11, wherein the control means is also configured to control the manipulator (10) in such a way that the pressing force is steadily increased, and wherein the manipulator system is configured to determine a maximum permissible pressing force based on a first-time actuation of the force reduction input means (30), specifically while the pressing force is being steadily increased.

13. Method for controlling a manipulator (10), wherein the manipulator (10) guides an instrument (11), comprising:
- pressing the instrument (11) against a human body (20) with a pressing force;
- during the pressing: detecting whether there is an actuation of a force reduction input means (30), wherein the input means (30) is provided separately from the manipulator (10) and is actuatable by an operator (20) for reduction of the pressing force, and acquiring measured values by means of the instrument (11);
- acquiring a quality of the measured values by means of a measured value evaluation means, wherein the measured value evaluation means is provided separately from the manipulator (10);
- controlling the manipulator (10) based on the acquired quality of the measured values, and if it is detected that there is a corresponding actuation of the force reduction input means (30): controlling the manipulator (10) in order to reduce the pressing force to an amount greater than zero.

14. Manipulator system according to one of Claims 1 to 12 or method according to Claim 13, wherein the instrument (11) is configured for imaging and in particular for tomography.

## Revendications

1. Système de manipulation comprenant :
- un manipulateur (10) conçu pour guider un instrument (11) ;
- un moyen de commande conçu pour commander le manipulateur (10), de sorte que l'instrument (11) soit pressé contre un corps humain (20) avec une force de pression ;
- un moyen d'entrée (30) de réduction de force prévu séparément du manipulateur (10) et apte à être actionné par un opérateur (20) pour réduire la force de pression ;
- le moyen de commande étant en outre agencé pour commander le manipulateur (10) de telle sorte que la force de pression soit réduite à une valeur supérieure à zéro, sur la base d'un actionnement correspondant du moyen d'entrée (30) de réduction de force, **caractérisé en ce que** :
- le système de manipulation est conçu pour enregistrer des valeurs de mesure au moyen de l'instrument (11) pendant le pressage ; **en ce que**
- le système de manipulation comprend en outre un moyen d'évaluation des valeurs de mesure, qui est agencé pour détecter une qualité des valeurs de mesure ; et **en ce que**
- le moyen de commande est en outre agencé pour commander le manipulateur (10) sur la base de la qualité détectée des valeurs de mesure.

2. Système de manipulation selon la revendication 1, dans lequel le moyen d'entrée (30) de réduction de force est une interface utilisateur comprenant un bouton, un commutateur ou une commande, qui est de préférence apte à être actionné manuellement.

3. Système de manipulation selon l'une des revendications 1 à 2, dans lequel le manipulateur (10) comprend des capteurs de force et/ou de couple, et dans lequel le moyen de commande est de préférence agencé pour commander le manipulateur (10) au moyen d'une régulation de force, de position ou d'une régulation hybride.

4. Système de manipulation selon l'une des revendications 1 à 3,
- dans lequel le moyen d'entrée (30) de réduction de force est en outre apte à être actionné par un opérateur (20) pour interrompre le pressage ;
- dans lequel le moyen de commande est en outre adapté pour commander le manipulateur (10) de telle sorte que la force de pression soit réduite à zéro et que l'instrument (11) soit retiré du corps (20) ou puisse être retiré manuellement sur la base d'un actionnement correspondant du moyen d'entrée (30) de réduction de force.

5. Système de manipulation selon l'une des revendications 1 à 4,
- dans lequel le moyen d'entrée (30) de réduction de force est apte à être actionné de manière analogue ;
- le moyen de commande étant agencé pour commander le manipulateur (10) de manière que la force de pression dépende d'un degré d'actionnement analogique correspondant du moyen d'entrée (30) de réduction de force.

6. Système de manipulation selon la revendication 5, dans lequel le moyen de commande est agencé pour commander le manipulateur (10) de telle sorte que la force de pression soit réduite proportionnellement ou de manière non linéaire à l'étendue de l'actionnement analogique correspondant du moyen d'entrée (30) de réduction de force ou sur la base d'une dérivée temporelle de l'étendue de l'actionnement analogique correspondant du moyen d'entrée (30) de réduction de force.

7. Système de manipulation selon l'une des revendications 1 à 6,
- dans lequel le moyen d'entrée (30) de réduction de force est apte à être actionné numériquement ;
- le moyen de commande étant agencé pour commander le manipulateur (10) de telle sorte que la force de pression soit réduite en continu pendant un actionnement numérique correspondant continu et/ou par étapes individuelles pendant un actionnement numérique correspondant répété et/ou d'un facteur prédéfini ou d'une valeur prédéfinie pendant un seul actionnement numérique correspondant.

8. Système de manipulation selon l'une des revendications 1 à 7, dans lequel le moyen d'entrée (30) de réduction de force est agencé pour fournir un retour haptique, de préférence par vibration, à l'opérateur (20) .

9. Système de manipulation selon l'une des revendications 1 à 8, dans lequel le moyen de commande est en outre agencé pour commander le manipulateur (10) de telle sorte qu'un mouvement de l'instrument (11) le long du corps (20) soit interrompu et/ou que l'instrument (11) soit éloigné du corps (20) lorsqu'il est déterminé par le système de manipulation que la force de pression réduite à une valeur supérieure à zéro est inférieure à une valeur limite de la force de pression.

10. Système de manipulation selon la revendication 1,
- dans lequel le moyen d'évaluation des valeurs de mesure est en outre conçu de façon à déterminer si la qualité détectée des valeurs de mesure est inférieure à une valeur limite de qualité ;
- dans lequel le moyen de commande est en outre conçu de façon à commander le manipulateur (10) de telle sorte qu'un mouvement de l'instrument (11) le long du corps (20) soit interrompu et/ou que l'instrument (11) soit éloigné du corps (20) lorsqu'il est constaté par le moyen d'évaluation des valeurs de mesure que la qualité détectée des valeurs de mesure est inférieure à la valeur limite de qualité.

11. Système de manipulation selon l'une des revendications 1 à 10, comprenant en outre :
- des capteurs pour surveiller les signes vitaux du corps (20) afin de déterminer un état de stress ;
- un moyen d'évaluation du stress qui est agencé afin de détecter un état de stress du corps (20) à l'aide des signes vitaux surveillés et pour déterminer si l'état de stress détecté dépasse une valeur limite de stress ;
- le moyen de commande étant en outre agencé pour commander le manipulateur (10) de telle sorte que la force de pression soit réduite à zéro et que l'instrument (11) soit retiré du corps (20) ou puisse être retiré manuellement lorsqu'il est déterminé par le moyen d'évaluation du stress que l'état de stress détecté dépasse la valeur limite de stress.

12. Système de manipulation selon l'une des revendications 1 à 11, dans lequel le moyen de commande est en outre agencé pour commander le manipulateur (10) de telle sorte que la force de pression soit augmentée de manière continue, et dans lequel le système de manipulation est agencé pour déterminer une force de pression maximale autorisée sur la base d'un premier actionnement du moyen d'entrée (30) de réduction de force, tout en augmentant de manière continue la force de pression.

13. Procédé de commande d'un manipulateur (10), dans lequel le manipulateur (10) guide un instrument (11), comprenant le fait de :
- presser l'instrument (11) contre un corps humain (20) avec une force de pression ;
- pendant le pressage : détecter s'il y a une action sur un moyen d'entrée (30) de réduction de force, le moyen d'entrée (30) étant prévu séparément du manipulateur (10) et étant apte à être actionné par un opérateur (20) de façon à réduire la force de pressage et à détecter des valeurs de mesure au moyen de l'instrument (11) ;
- saisir une qualité des valeurs de mesure avec un moyen d'évaluation des valeurs de mesure, le moyen d'évaluation des valeurs de mesure étant prévu séparément du manipulateur (10) ;
- commander le manipulateur (10) sur la base de la qualité détectée des valeurs mesurées et lorsqu'il est détecté qu'il existe un actionnement correspondant du moyen d'entrée (30) de réduction de force : commande du manipulateur (10) pour réduire la force de pression à une valeur supérieure à zéro.

14. Système de manipulation selon l'une des revendications l à 12 ou procédé selon la revendication 13, dans lequel l'instrument (11) est agencé pour l'imagerie et notamment la tomographie.
